# EUROPEAN PATENT APPLICATION

(11) **EP 4 664 467 A1**
(43) Date of publication of application: **17.12.2025**
(21) Application number: 25180361.5
(22) Date of filing: 03.06.2025
(51) Int. Cl.: G16H 10/60, G16H 15/00, G16H 50/70, G16H 80/00

(54) **SYSTEM AND COMPUTER-IMPLEMENTED METHOD FOR GENERATING CLINICAL NOTES**

(30) Priority: 11.06.2024 US 202463658578 P
(71) Applicant: Solventum Intellectual Properties Company, Maplewood, MN 55144 (US)
(72) Inventor: Schaaf, Thomas, Pittsburgh, 15208 (US); Fancellu, Federico, Dublin, A94W8R6 (IE); Zhang, Longxiang, Pittsburgh, 15206 (US); Koll, Detlef, Pittsburgh, 15208 (US)
(74) Representative: Vossius & Partner Patentanwälte Rechtsanwälte mbB

(57) **Abstract**

A system includes a non-transitory storage having stored thereon instructions that when executed by a processor cause the processor to train a machine learning model to generate clinical notes by repeatedly: receiving a plurality of clinical notes associated with a patient; selecting a final clinical note; selecting a predecessor clinical note chronologically preceding the final clinical note; determining overlapping data between the final clinical note and the predecessor clinical note; performing an action on the identified overlapping data to generate a synthesized input clinical note; providing the synthesized input clinical note and the predecessor clinical note to the machine learning model; receiving a predicted final clinical note from the machine learning model; and updating the machine learning model based on the predicted final clinical note and the final clinical note.

## Description

### Technical Field

The present disclosure relates generally to a system and a computer-implemented method for generating clinical notes, and more specifically to a system and a computer-implemented method for training a machine learning model to generate clinical notes and using the trained machine learning model to generate augmented clinical notes.

### Background

Clinical notes are typically generated based on a conversation between a doctor and a patient, i.e., a doctor-patient-conversation or "DoPaCo." However, the clinical notes that are generated solely based on the DoPaCo may lack important clinical information related to the patient that was recorded during previous interactions with the patient.

Such important clinical information related to the patient may be present in historical clinical notes associated with the patient. Typically, the historical clinical notes are stored in an electronic health record (EHR). It may be desirable that clinical notes also include the important clinical information that is present in the historical clinical notes in addition to information based on the DoPaCo.

Therefore, there is a need for a system and a computer-implemented method for generating improved clinical notes based upon DoPaCo that include the important clinical information present in the historical clinical notes associated with the patient.

### Summary

In a first aspect, the present disclosure provides a system. The system includes one or more computer processors. The system further includes a non-transitory computer-readable storage having stored thereon instructions that when executed by the one or more processors cause the one or more processors to train a machine learning model to generate clinical notes by repeatedly: receiving a plurality of clinical notes associated with a patient; selecting a final clinical note from the plurality of clinical notes; selecting a predecessor clinical note from the plurality of clinical notes, where the predecessor clinical note chronologically precedes the final clinical note; determining overlapping data between the final clinical note and the predecessor clinical note; identifying the overlapping data from the final clinical note; performing an action on the identified overlapping data from the final clinical note to generate a synthesized input clinical note, such that the synthesized input clinical note is devoid of the overlapping data; providing the synthesized input clinical note and the predecessor clinical note to the machine learning model; receiving a predicted final clinical note from the machine learning model, where the machine learning model generates the predicted final clinical note based on the synthesized input clinical note and the predecessor clinical note; and updating the machine learning model based on the predicted final clinical note and the final clinical note. The instructions when executed by the one or more processors further cause the one or more processors to output the machine learning model to at least one of a storage device or the non-transitory computer-readable storage.

In a second aspect, the present disclosure provides a computer-implemented method for using a machine learning model to generate clinical notes. The computer-implemented method includes training the machine learning model to generate clinical notes by repeatedly: receiving a plurality of clinical notes associated with a patient; selecting a final clinical note from the plurality of clinical notes; selecting a predecessor clinical note from the plurality of clinical notes, where the predecessor clinical note chronologically precedes the final clinical note; determining overlapping data between the final clinical note and the predecessor clinical note; identifying the overlapping data from the final clinical note; performing an action on the identified overlapping data from the final clinical note to generate a synthesized input clinical note, such that the synthesized input clinical note is devoid of the overlapping data; providing the synthesized input clinical note and the predecessor clinical note to the machine learning model; receiving a predicted final clinical note from the machine learning model, where the machine learning model generates the predicted final clinical note based on the synthesized input clinical note and the predecessor clinical note; and updating the machine learning model based on the predicted final clinical note and the final clinical note. The computer-implemented method further includes outputting the machine learning model to at least one of a storage device or a non-transitory computer-readable storage.

The details of one or more examples of the disclosure are set forth in the accompanying drawings and the description below. Other features, objects, and advantages of the disclosure will be apparent from the description and drawings, and from the claims.

### Brief Description of the Drawings

Exemplary embodiments disclosed herein may be more completely understood in consideration of the following detailed description in connection with the following figures. The figures are not necessarily drawn to scale. Like numbers used in the figures refer to like components. However, it will be understood that the use of a number to refer to a component in a given figure is not intended to limit the component in another figure labeled with the same number.
FIG. 1 is a schematic block diagram of a system according to an embodiment of the present disclosure;
FIG. 2 is a schematic diagram of a clinical note according to an embodiment of the present disclosure;
FIG. 3A is a schematic diagram of a predecessor clinical note according to an embodiment of the present disclosure;
FIG. 3B is a schematic diagram of a final clinical note according to an embodiment of the present disclosure;
FIG. 3C is a schematic diagram of a synthesized clinical note according to an embodiment of the present disclosure;
FIG. 4 is a schematic diagram of a predicted final clinical note according to an embodiment of the present disclosure;
FIG. 5 is a schematic diagram of a current clinical note according to an embodiment of the present disclosure;
FIG. 6 is a schematic diagram of an augmented clinical note according to an embodiment of the present disclosure;
FIG. 7 is a flowchart depicting various steps of a computer-implemented method according to an embodiment of the present disclosure;
FIG. 8 is a flowchart depicting various further steps of the computer-implemented method according to an embodiment of the present disclosure; and
FIG. 9 is a flowchart depicting various steps of a process according to an embodiment of the present disclosure.

### Detailed Description

In the following description, reference is made to the accompanying figures that form a part thereof and in which various embodiments are shown by way of illustration. It is to be understood that other embodiments are contemplated and may be made without departing from the scope or spirit of the present disclosure. The following detailed description, therefore, is not to be taken in a limiting sense.

In the present disclosure, the following definitions are adopted.

As recited herein, all numbers should be considered modified by the term "about." As used herein, "a," "an," "the," "at least one," and "one or more" are used interchangeably.

As used herein as a modifier to a property or attribute, the term "generally," unless otherwise specifically defined, means that the property or attribute would be readily recognizable by a person of ordinary skill but without requiring absolute precision or a perfect match (e.g., within +/- 20 % for quantifiable properties).

The term "substantially," unless otherwise specifically defined, means to a high degree of approximation (e.g., within +/- 10% for quantifiable properties) but again without requiring absolute precision or a perfect match.

The term "about," unless otherwise specifically defined, means to a high degree of approximation (e.g., within +/- 5% for quantifiable properties) but again without requiring absolute precision or a perfect match.

Terms such as same, equal, uniform, constant, strictly, and the like, are understood to be within the usual tolerances or measuring error applicable to the particular circumstance rather than requiring absolute precision or a perfect match.

As used herein, when a first material is termed as "similar" to a second material, at least 90% by weight of the first and second materials are identical and any variation between the first and second materials comprises less than about 10% by weight of each of the first and second materials.

As used herein, "at least one of A and B" and "at least one of A or B" should be understood to mean "only A, only B, or both A and B."

As used herein, the term "processor" or "computer processor" refers any device that performs logic operations. A processor may include a general processor, a central processing unit, an application specific integrated circuit (ASIC), a digital signal processor, a field programmable gate array (FPGA), a digital circuit, an analog circuit, a controller, a microcontroller, any other type of processor, or any combination thereof.

As used herein, the term "instructions" refers to code (e.g., source code, compiled code, code that can be interpreted, executable code, etc.) that, when executed by a processor, causes the processor to perform various steps, functions, operations, and/or calculations, i.e., the conventional meaning of the term "instructions" with respect to digital technology.

As used herein, the term "storage device" refers to any storage medium that is capable of storing data and information in an electronic format. Examples of a storage device include hard drives, flash drives, optical media, and the like.

As used herein, the term "communicably coupled" refers to any coupling that allows exchange of data via a communication medium. Two communicably coupled components may be electrically coupled by, for example, a wire; optically coupled by, for example, an optical cable; and/or wirelessly coupled by, for example, a radio frequency or other transmission media. Two communicably coupled components may be directly coupled, or indirectly coupled, such as via a network.

As used herein, the terms "medical" and "clinical" are interchangeable and have the same meaning.

As used herein, the term "doctor" or "physician" broadly refers to a health care provider or a medical professional.

As used herein, the term "patient," and its equivalents, refers to an individual being monitored and/or cared for within a clinical environment or who has been previously monitored and/or cared for within the clinical environment. In various examples, a patient is a human, but implementations of this disclosure are not limited thereto. Examples of the clinical environment may include, but are not limited to, a doctor's office, a medical facility, a medical practice, a medical lab, an urgent care facility, a medical clinic, an emergency room, an operating room, a hospital, a long term care facility, a rehabilitation facility, a nursing home, and a hospice facility.

As used herein, the term "user" refers to, for example, a physician (including, but not limited to, a radiologist, a surgeon, a primary care physician, and a medical specialist), a physician assistant, a medical scribe, a nursing professional, a medical laboratory technician, medical clinics, hospitals, health insurance providers, diagnostic sites, imaging sites, pharmacies, and the like. The term "user" may also refer to an academic institution, a government research laboratory, a non-profit entity, or a for-profit entity, such as a pharmaceutical, health insurance, biotechnology, wearable device, physiological monitoring, or medical device company.

As used herein, the term "clinical note" refers to a note including clinical data of a patient that is generated based on an interaction between the patient and a medical professional. Clinical notes may be stored in an electronic format (e.g., a text document), typically in an electronic health record (EHR).

As used herein, the term "clinical data" refers to data describing an individual person's medical history or medical condition, including lab test results, medication history, immunization history, and so forth.

As used herein, the term "electronic health record" or "EHR" refers to a database that stores clinical notes and other clinical data related to a patient. EHR may be queried to receive the clinical notes and the other clinical data.

As used herein, the term "audio recording" refers to audio data stored in an electronic format (e.g., MP3, AAC).

As used herein, the term "machine learning model" or "ML model" refers to a machine learning algorithm or collection of algorithms that takes structured and/or unstructured data inputs and generates a prediction or result. That is, a machine learning model may be a computer model or a computer representation that may be tuned (e.g., trained) based on inputs to approximate unknown functions. The process of building or optimizing a machine learning model is referred to herein as "training." Examples of machine-learning models include, for example, one or more of vectorization machine-learning models, sequence-to-sequence models, transformer models, a decision tree (e.g., a gradient boosted decision tree), a linear regression model, a logistic regression model, association rule learning, inductive logic programming, support vector learning, a Bayesian network, a regression-based model, a neural network, or combinations thereof.

As used herein, the term "clinical metadata" refers to the data that provides information about one or more aspects of a clinical note. For example, clinical metadata may provide information regarding the creation date of the clinical note and the document type of the clinical note.

As used herein, the term "document type" refers to the type or category that a clinical note falls into.

As used herein, the term "clinical concept" represents any attribute of a patient. Such attributes include, but are not limited to, a chief complaint of the patient, a history of present illness of the patient, a past medical history of the patient, a social history of the patient, a family history of the patient, a review of systems of the patient, allergies of the patient, medications of the patient, impressions of the patient by a clinician, a medical plan for the patient, diagnostic imaging results preformed the patient, results of a medical test of the patient, a gender of the patient, an ethnicity of the patient, an age of the patient, a physical attribute of the patient, physical signs of the patient, physical systems of the patient, a time period associated with one of the preceding attributes or another attribute, and/or other attributes.

As used herein, the term "embedding" refers to a mathematical representation of a set of data points in a lower-dimensional space that captures their underlying relationships and patterns. Embeddings are often used to represent complex data types, such as images, text, or audio, in a way which machine learning algorithms can easily process. Embeddings may be a numerical or vector representation of a variable. One example of an embedding is a token embedding. In the context of the present disclosure, embeddings encompass additive combination of text embeddings and concept embeddings.

The present disclosure provides a system. The system includes one or more computer processors. The system further includes a non-transitory computer-readable storage having stored thereon instructions that when executed by the one or more processors cause the one or more processors to train a machine learning model to generate clinical notes by repeatedly: receiving a plurality of clinical notes associated with a patient; selecting a final clinical note from the plurality of clinical notes; selecting a predecessor clinical note from the plurality of clinical notes, where the predecessor clinical note chronologically precedes the final clinical note; determining overlapping data between the final clinical note and the predecessor clinical note; identifying the overlapping data from the final clinical note; performing an action on the identified overlapping data from the final clinical note to generate a synthesized input clinical note, such that the synthesized input clinical note is devoid of the overlapping data; providing the synthesized input clinical note and the predecessor clinical note to the machine learning model; receiving a predicted final clinical note from the machine learning model, where the machine learning model generates the predicted final clinical note based on the synthesized input clinical note and the predecessor clinical note; and updating the machine learning model based on the predicted final clinical note and the final clinical note. The instructions when executed by the one or more processors further cause the one or more processors to output the machine learning model to at least one of a storage device or the non-transitory computer-readable storage.

The system of the present disclosure may train the machine learning model using the plurality of clinical notes associated with the patient to generate clinical notes, or more specifically, augmented clinical notes.

Particularly, after training, the machine learning model may receive a current clinical note (i.e., a clinical note generated based on doctor-patient-conversation (DoPaCo)) and a plurality of historical notes and generate an augmented clinical note based on the current clinical note. That is, the plurality of clinical notes and the current clinical note may be inputs to the machine learning model, and the augmented clinical note may be an output from the machine learning model.

The augmented clinical note generated by the machine learning model may include historical clinical data related to the patient that is not present in the current clinical note. In other words, the augmented clinical note may include important clinical data related to the patient that is missing from the current clinical note. Specifically, the machine learning model may learn to select appropriate historical clinical data from the plurality of clinical notes to generate the augmented clinical note. In some examples, the machine learning model may also modify (e.g., correct) clinical data of the current clinical note based on the training, and output the modified clinical data in the augmented clinical note.

In some cases, the machine learning model may flag the historical clinical data and the modified clinical data in the augmented clinical note for review by the user. This may allow the user to double-check whether the historical clinical data and the modified clinical data in the augmented clinical note are correct. Moreover, upon review, if the user makes corrections to the augmented clinical note, the augmented clinical note with the corrections may be used to further train the machine learning model.

The system may therefore facilitate generation of clinical notes with more complete information (e.g., historical information related to the patient) and correct information than what is present in the current clinical note.

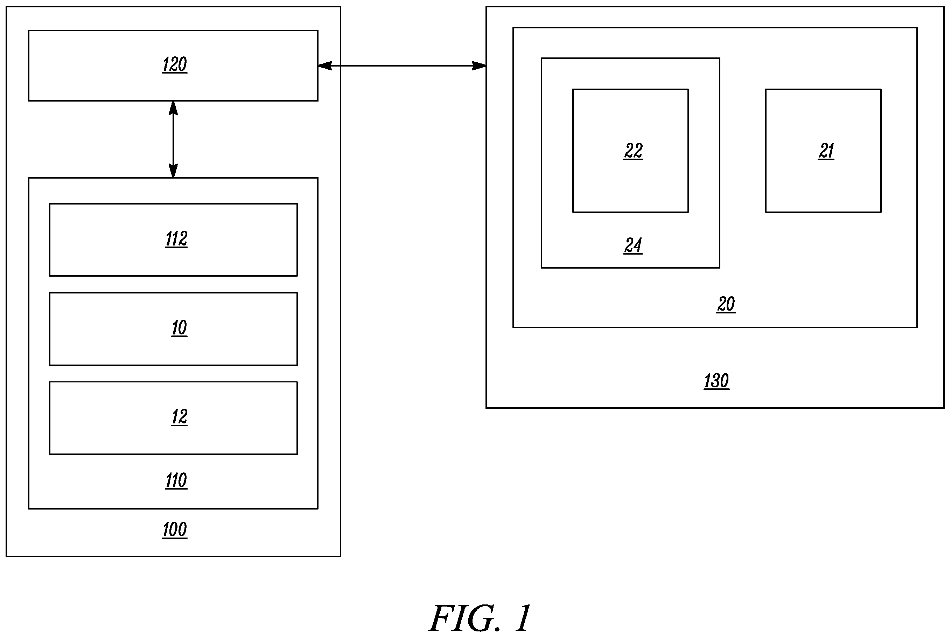

Referring now to the figures, FIG. 1 illustrates a schematic block diagram of a system 100 according to an embodiment of the present disclosure.

The system 100 includes a non-transitory computer-readable storage 110 (hereinafter referred to as "the non-transitory storage 110") having stored thereon instructions 112. That is, the instructions 112 are stored on the non-transitory storage 110.

The system 100 further includes one or more computer processors 120 (hereinafter referred to as "the processor 120"). The processor 120 may be communicably coupled to the non-transitory storage 110. The processor 120 may be further communicably coupled to a storage device 130.

The storage device 130 may store a plurality of clinical notes 20 associated with a patient. Specifically, the storage device 130 may store an electronic health record (EHR) that includes the plurality of clinical notes 20 associated with the patient. The processor 120 may access the storage device 130, or more specifically, the EHR stored on the storage device 130, to receive the plurality of clinical notes 20 associated with the patient.

Clinical notes refer to notes that are generated to record information about the patient and are typically in text, i.e., unstructured, format. Clinical notes may be of various types, including, but not limited to, progress notes, admission notes, discharge notes, history and physical notes, and consultation notes.

Clinical notes may include clinical data, such as, for example, chief complaint (CC), history of present illness (HPI), vital signs (VS), review of systems (ROS), physical examination, results (i.e., information related to lab results or imaging scans), assessment or impressions/diagnosis, plan (i.e., treatment recommendations), and orders (i.e., information about prescriptions, procedures, tests, referrals to specialists, and follow-up care).

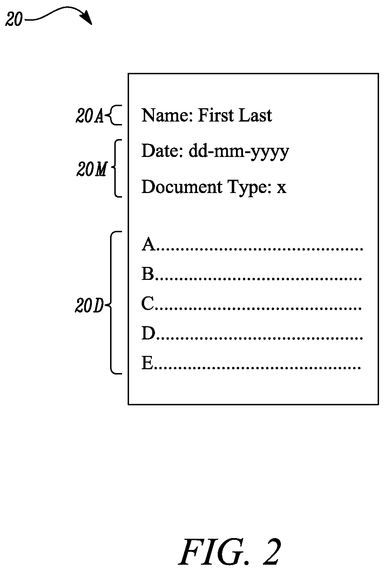

Referring to FIG. 2, a clinical note 20 from the plurality of clinical notes 20 is shown. Each clinical note 20 from the plurality of clinical notes 20 may include general data 20A that is related to the patient. The general data 20A may include, for example, name of the patient, date of birth of the patient, age of the patient, gender of the patient, and the like. The general data 20A may facilitate querying the storage device 130, or more specifically, the EHR stored on the storage device 130, for the plurality of clinical notes 20 associated with the patient.

Each clinical note 20 may further include clinical data 20D. The clinical data 20D may be recorded during treatment of the patient. The clinical data 20D may be of any type, such as any of the above-described examples of clinical data. One or more clinical concepts may be extracted from the clinical data 20D. In some examples, the one or more clinical concepts may be automatically extracted from the clinical data 20D by use of natural language understanding (NLU). In some cases, the one or more clinical concepts may be manually extracted from the clinical data 20D. Each clinical note 20 may include the one or more clinical concepts in the form of embeddings.

Each clinical note 20 may further include clinical metadata 20M. In some embodiments, the clinical metadata 20M may include one or more of a document type and a creation date. In some examples, the clinical metadata 20M may further include a physician name.

While the clinical metadata 20M is depicted as text in FIG. 2 for illustrative purposes, it may be noted that each clinical note 20 may include the clinical metadata 20M in the form of embeddings. Further, as will be described below, the plurality of clinical notes 20 may be used to train a machine learning model 10 (shown in FIG. 1) to generate clinical notes. Therefore, in some embodiments, each of the plurality of clinical notes 20 may be embedded with the clinical metadata 20M prior to training of the machine learning model 10. The clinical metadata 20M may facilitate training of the machine learning model 10. Additionally, the one or more clinical concepts embedded in each clinical note 20 may further facilitate training of the machine learning model 10.

Referring now to FIGS. 1 and 2, the instructions 112 when executed by the processor 120 cause the processor 120 to train the machine learning model 10 to generate clinical notes by repeatedly performing the following steps.

The steps include receiving the plurality of clinical notes 20 associated with the patient. The processor 120 may communicate with the storage device 130 to receive the plurality of clinical notes 20.

The steps further include selecting a final clinical note 21 from the plurality of clinical notes 20. In some embodiments, the processor 120 may select the latest clinical note (i.e., the clinical note 20 having a latest creation date) from the plurality of clinical notes 20 as the final clinical note 21.

The steps further include selecting a predecessor clinical note 22 from the plurality of clinical notes 20. The predecessor clinical note 22 chronologically precedes the final clinical note 21. That is, the predecessor clinical note 22 has a creation date earlier than that of the final clinical note 21.

In some embodiments, each clinical note 20 having a creation date earlier than that of the final clinical note 21 may be determined based on the clinical metadata 20M. Specifically, in some embodiments, selecting the predecessor clinical note 22 may include determining a creation date of the final clinical note 21. In some embodiments, selecting the predecessor clinical note 22 may further include determining a plurality of historical clinical notes 24 from the plurality of clinical notes 20 that chronologically precedes the final clinical note 21, such that each of the plurality of historical clinical notes 24 has a creation date earlier than the creation date of the final clinical note 21.

In some embodiments, selecting the predecessor clinical note 22 may further include selecting a historical clinical note 24 from the plurality of historical clinical notes 24 as the predecessor clinical note 22 if a difference between the creation date of the historical clinical note 24 and the creation date of the final clinical note 21 is less than a predetermined time period. That is, a historical clinical note 24 from the plurality of historical clinical notes 24 may be selected as the predecessor clinical note 22 only if the difference between the creation date of the historical clinical note 24 and the creation date of the final clinical note 21 is less than the predetermined time period. This may prevent selection of a clinical note 20 that has an exceedingly earlier creation date than the creation date of the final clinical note 21 as the predecessor clinical note 22, which may improve training of the machine learning model 10.

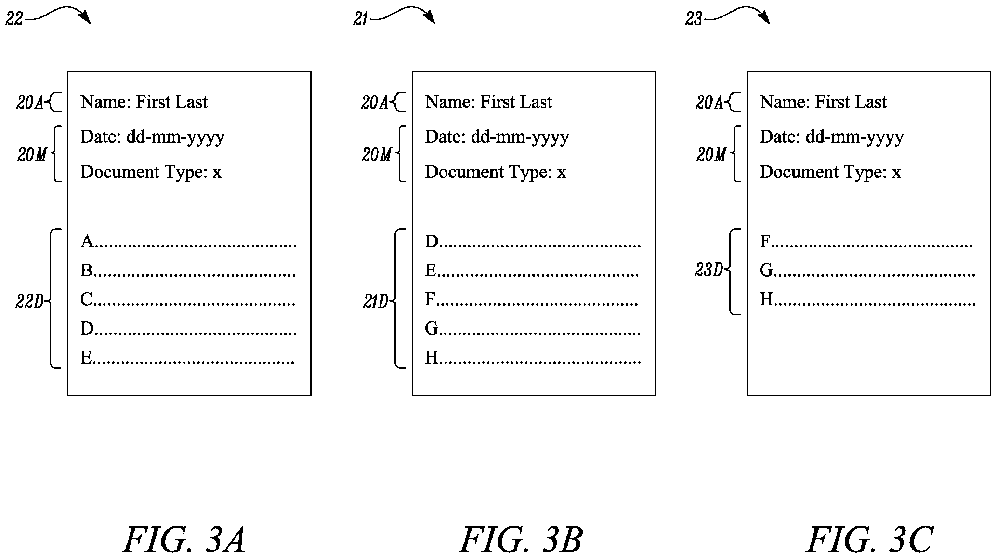

An example of the predecessor clinical note 22 is shown in FIG. 3A, and an example of the final clinical note 21 is shown in FIG. 3B.

Referring to FIGS. 1, 3A, and 3B, the steps further include determining overlapping data between the final clinical note 21 and the predecessor clinical note 22. The overlapping data between the final clinical note 21 and the predecessor clinical note 22 may be determined by comparing the final clinical data 21D of the final clinical note 21 and predecessor clinical data 22D of the predecessor clinical note 22. In some implementations, the overlapping data may refer to portions of the predecessor clinical note 22 that are in some way reflected in the final clinical note 21. For instance, the overlapping data may pertain to medical concepts, patient vitals, lab results, physician findings, and other medically relevant information. The overlapping data may include replicated information (e.g., generated using conventional copy-and-paste techniques), paraphrased information, or some combination of the two. By way of example, the replicated information can be identified as the overlapping data by using a string comparison or other comparison technique that evaluates the similarity between information and recognizing information above a threshold similarity score as the overlapping data. The paraphrased information can be identified as the overlapping data by comparing sections of text to identify substantially similar constraints in close proximity to the compared section of text. For instance, a description for a particular medication may be identified as paraphrased overlapping data in the final clinical note 21 (even if it is not substantially similar to the portion of text in the predecessor clinical note 22) if the description is in close proximity to a name for the medication or a dosage of the medication that are in the final clinical note 21 substantially similar to text in the predecessor clinical note 22.

In some embodiments, a model 12 may determine the overlapping data between the final clinical note 21 and the predecessor clinical note 22. In some examples, the model 12 may be a pre-trained machine learning model. The model 12 may be pre-trained using synthetic data and/or real data to determine the overlapping data. In some other examples, the model 12 may be a rule-based model.

In some embodiments, the determination of the overlapping data between the final clinical note 21 and the predecessor clinical note 22 is performed without access to historical electronic health record (EHR) data associated with the patient. The historical EHR data may refer to historical data associated with the patient that is stored in the EHR apart from the plurality of clinical notes 20. Therefore, the overlapping data may be determined solely based on the final clinical note 21 and the predecessor clinical note 22.

The steps further include identifying the overlapping data from the final clinical note 21.

The steps further include performing an action on the identified overlapping data from the final clinical note 21 to generate a synthesized input clinical note 23 (shown in FIG. 3C), such that the synthesized input clinical note 23 is devoid of the overlapping data. Specifically, the synthesized input clinical note 23 may include synthesized clinical data 23D.

In some embodiments, performing the action on the identified overlapping data includes removing the identified overlapping data from the final clinical note 21. Therefore, the synthesized clinical data 23D may include the final clinical data 21D devoid of the overlapping data between the final clinical note 21 and the predecessor clinical note 22. By way of explanation, if set A contains the final clinical data 21D, set B contains the predecessor clinical data 22D, then the synthesized clinical data 23D may be defined as A - B.

The steps further include providing the synthesized input clinical note 23 and the predecessor clinical note 22 to the machine learning model 10. That is, the synthesized input clinical note 23 and the predecessor clinical note 22 may be input to the machine learning model 10.

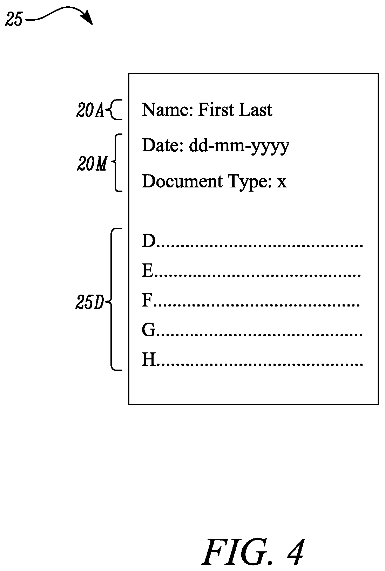

The steps further include receiving a predicted final clinical note 25 (shown in FIG. 4) from the machine learning model 10. The machine learning model 10 generates the predicted final clinical note 25 based on the synthesized input clinical note 23 and the predecessor clinical note 22.

The final clinical note 21 may be a training target for the machine learning model 10. As a result, it may be desirable that the predicted final clinical note 25 generated by the machine learning model 10 is equivalent to the final clinical note 21. Specifically, it may be desirable that predicted final clinical data 25D of the predicted final clinical note 25 is equivalent to the final clinical data 21D of the final clinical note 21.

The steps further include updating the machine learning model 10 based on the predicted final clinical note 25 and the final clinical note 21.

In some embodiments, the steps may further include selecting another clinical note 20 from the plurality of clinical notes 20, if present, as the predecessor clinical note 22 and repeating the aforementioned steps to train the machine learning model 10. For example, if there are n-number of the plurality of clinical notes 20 that can be selected as the predecessor clinical note 22 with respect to the final clinical note 21 (i.e., each of them chronically precedes the final clinical note 21), the steps may include iteratively selecting each of the n-number of the plurality of clinical notes 20 as the predecessor clinical note 22 for training the machine learning model 10.

Moreover, in some embodiments, the processor 120 may further select another clinical note 20 from the plurality of clinical notes 20, if present, as the final clinical note 21. Accordingly, if there are m-number of the plurality of clinical notes 20 that can be selected as the predecessor clinical note 22 with respect to the final clinical note 21, the steps may further include iteratively selecting each of the m-number of the plurality of clinical notes 20 as the predecessor clinical note 22 for training the machine learning model 10.

In other words, a suitable pair of clinical notes 20 (one as the final clinical note 21 and the other as the predecessor clinical note 22) may be iteratively selected from the plurality of clinical notes 20 to train the machine learning model 10, such that each of the suitable pair of clinical notes 20 is used to train the machine learning model 10. In this way, all of the plurality of clinical notes 20 may be used to train the machine learning model 10. The processor 120 may repeatedly perform the aforementioned steps until training data (i.e., all of the plurality of clinical notes 20) is exhausted. The plurality of clinical notes 20 may therefore be interchangeably referred to as "the training data" for the machine learning model 10.

The aforementioned steps for training the machine learning model 10 may be particularly useful in case the storage device 130, or more specifically, the EHR stored on the storage device 130, does not have a large number of clinical notes 20 associated with the patient stored thereon, i.e., in case of data scarcity.

The instructions 112 when executed by the processor 120 further cause the processor 120 to output the machine learning model 10 to at least one of the storage device 130 or the non-transitory storage 110. In other words, the processor 120 may output the machine learning model 10, once trained using the plurality of clinical notes 20, to at least one the storage device 130 or the non-transitory storage 110.

Now referring to FIGS. 1, 5, and 6, in some embodiments, the instructions 112 when executed by the processor 120 further cause the processor 120 to receive a current clinical note 26 associated with the patient after training of the machine learning model 10. Any suitable input device (e.g., a mouse, a keyboard, etc.) may be utilized to provide the current clinical note 26 to the processor 120. The current clinical note 26 may include the general data 20A, the clinical metadata 20M, and current clinical data 26D (alternatively referred to as "the current data 26D").

The current clinical note 26 may include a transcription of a conversation (also referred to as "Doctor-Patient-Conversation" or "DoPaCo") between the patient and a user (e.g., a medical professional). In some embodiments, the transcription of the conversation may be manually performed by a scribe. In some other embodiments, the transcription of the conversation may be automatically performed by employing an automatic speech recognition technique. For example, an audio recording of the conversation may be automatically transcribed (e.g., converted to a textual representation) using the automatic speech recognition technique.

In some embodiments, the transcription may be pre-processed, such that the processor 120 receives the current clinical note 26 that includes the general data 20A, the clinical metadata 20M, and the current clinical data 26D. In some other embodiments, the processor 120 may receive and process the transcription to identify the general data 20A, the clinical metadata 20M, and the current clinical data 26D based on the transcription, and accordingly generate the current clinical note 26. In some examples, the processor 120 may also remove uninformative or redundant data from the transcription. The processor 120 may employ natural language processing (NLP), natural language understanding (NLU), natural language generation (NLG), machine learning models, or combinations thereof to generate the current clinical note 26 based on the transcription.

The instructions 112 when executed by the processor 120 may further cause the processor 120 to provide the current clinical note 26 to the machine learning model 10. The instructions 112 when executed by the processor 120 may further cause the processor 120 to receive an augmented clinical note 27 from the machine learning model 10. The machine learning model 10 may generate the augmented clinical note 27 based on the current clinical note 26. Therefore, the augmented clinical note 27 may include augmented clinical data 27D based at least on the current data 26D of the current clinical note 26.

Specifically, the augmented clinical note 27 may include the current data 26D from the current clinical note 26 and historical data 27H associated with the patient that is not present in the current clinical note 26. More specifically, the augmented clinical data 27D of the augmented clinical note 27 may include the current data 26D and the historical data 27H. The machine learning model 10 may incorporate the historical data 27H to at least a portion of the current data 26D in the augmented clinical note 27. The machine learning model 10 may incorporate the historical data 27H in the augmented clinical note 27 based on the learning during the training.

For example, if the machine learning model 10 learned that the patient has Type II diabetes during the training, and the current clinical note 26 (or the current data 26D) only specifies diabetes without its type, the machine learning model 10 may incorporate the historical data 27H (e.g., that the patient has Type II diabetes) in the augmented clinical note 27. As another example, if the current clinical note 26 (or the current data 26D) only specifies medication related to diabetes, the machine learning model 10 may incorporate information relevant to diabetes (e.g., that the patient has Type II diabetes) in the augmented clinical note 27.

In some embodiments, the machine learning model 10 may further flag the historical data 27H in the augmented clinical note 27 for review by the user. This may allow the user to double-check whether the historical data 27H incorporated by the machine learning model 10 is correct.

In some embodiments, the machine learning model 10 may modify at least a portion 27P of the current data 26D. The machine learning model 10 may modify the portion 27P of the current data 26D based on the learning during the training. For example, if the machine learning model 10 learned that the patient has Type II diabetes during the training, and if the current clinical note 26 (or the current data 26D) incorrectly specifies that the patient has Type I diabetes (i.e., incorrect diabetes type), the machine learning model 10 may modify the incorrect portion of the current data 26D with the correct data (i.e., the patient has Type II diabetes in this example).

The machine learning model 10 may further flag the portion 27P in the augmented clinical note 27 for review by the user. This may allow the user to double-check whether the portion 27P modified by the machine learning model 10 is correct.

It may be noted that the machine learning model 10 may flag the historical data 27H and/or the portion 27P only if the machine learning model 10 has low confidence (i.e., below a predefined threshold) associated with the historical data 27H and/or the portion 27P. Therefore, the machine learning model 10 may output the augmented clinical note 27 without flagging the historical data 27H and/or the portion 27P if the machine learning model 10 has high confidence (i.e., above the predefined threshold) associated with the historical data 27H and/or the portion 27P.

Further, it may be noted that the historical data 27H and the portion 27P of the current data 26D may be flagged by any suitable method or technique. For example, such data may be flagged by an actual flag being displayed next to the data, by underlining the data, by providing an undulating underline for the data, by highlighting the data, by bolding the data, by italicizing the data, etc.

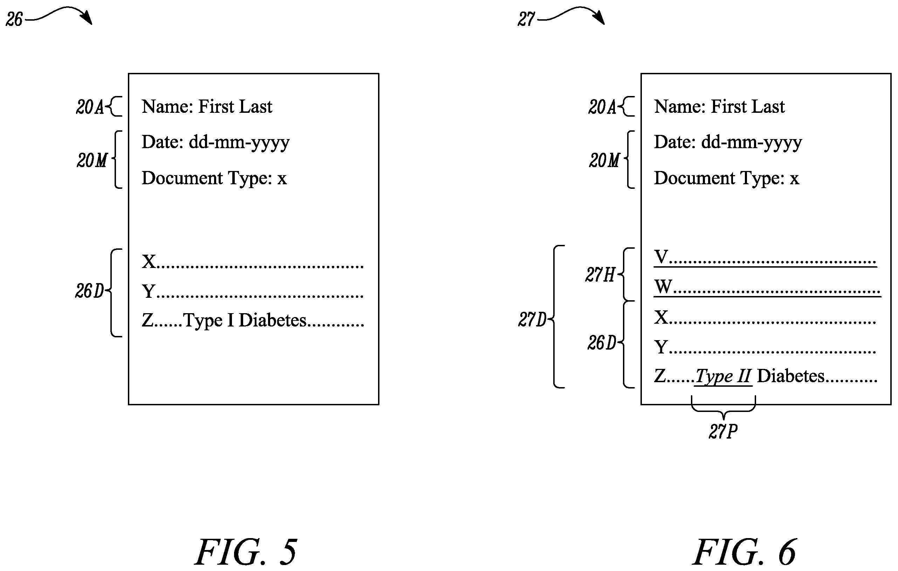

In some embodiments, the historical data 27H and the portion 27P of the current data 26D may be flagged in different manners to ensure that the user can distinguish between the historical data 27H and the portion 27P. For example, as shown in FIG. 6, solid underlines may be used to flag the historical data 27H and italicization may be used to flag the portion 27P of the current data 26D that is modified in the augmented clinical note 27.

The instructions 112 when executed by the processor 120 may further cause the processor 120 to output the augmented clinical note 27 to the user. The augmented clinical note 27 may be outputted to the user via any suitable output device. For example, the augmented clinical note 27 may be outputted to the user via a display device (such as a monitor).

As discussed above, the user may review the augmented clinical note 27. Therefore, the augmented clinical note 27 that is outputted to the user may be editable. That is, the user may review the augmented clinical note 27, and edit the augmented clinical note 27 if required. The augmented clinical note 27, if edited, may be provided to the machine learning model 10 for further training.

The system 100 may train the machine learning model 10 using the plurality of clinical notes 20 associated with the patient to generate clinical notes, or more specifically, augmented clinical notes. Particularly, after training, the machine learning model 10 may receive the current clinical note 26 and generate the augmented clinical note 27 based on the current clinical note 26. That is, the plurality of clinical notes 20 and the current clinical note 26 may be inputs to the machine learning model 10, and the augmented clinical note 27 may be an output from the machine learning model 10.

The augmented clinical note 27 generated by the machine learning model 10 may include historical clinical data related to the patient that is not present in the current clinical note 26. In other words, the augmented clinical note 27 may include important clinical data related to the patient that is missing from the current clinical note 26. Specifically, the machine learning model 10 may learn to select appropriate historical clinical data from the plurality of clinical notes 20 to generate the augmented clinical note 27. In some examples, the machine learning model 10 may also modify (e.g., correct) clinical data of the current clinical note 26 based on the training, and output the modified clinical data (e.g., the portion 27P) in the augmented clinical note 27.

In some cases, the machine learning model 10 may flag the historical clinical data and the modified clinical data in the augmented clinical note 27 for review by the user. This may allow the user to double-check whether the historical clinical data and the modified clinical data in the augmented clinical note 27 are correct. Moreover, upon review, if the user makes corrections to the augmented clinical note 27, the augmented clinical note 27 with the corrections may be used to further train the machine learning model 10.

The system 100 may therefore facilitate generation of clinical notes with more complete (e.g., historical information related to the patient) and correct information than what is present in the current clinical note 26.

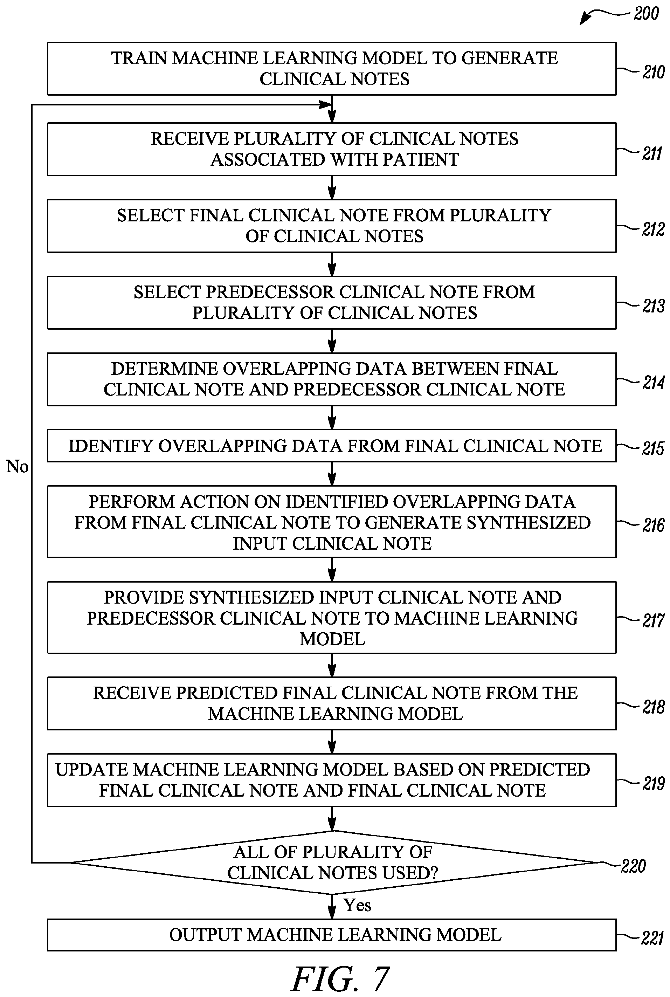

FIG. 7 illustrates a flowchart depicting various steps of a computer-implemented method 200 (hereinafter referred to as "the method 200") for using a machine learning model to generate clinical notes according to an embodiment of the present disclosure. The method 200 may be performed by the processor 120 of the system 100 of FIG. 1. The method 200 will be described with additional reference to FIGS. 1-6.

At step 210, the method 200 includes training the machine learning model to generate clinical notes by repeatedly performing steps 211-219. Referring to FIG. 1, for example, the method 200 may include training the machine learning model 10 to generate clinical notes by repeatedly performing steps 211-219.

At step 211, the method 200 further includes receiving a plurality of clinical notes associated with a patient. Referring to FIG. 1, for example, the method 200 may include receiving the plurality of clinical notes 20 associated with the patient.

At step 212, the method 200 further includes selecting a final clinical note from the plurality of clinical notes. Referring to FIGS. 1 and 3B, for example, the method 200 may include selecting the final clinical note 21 from the plurality of clinical notes 20.

At step 213, the method 200 further includes selecting a predecessor clinical note from the plurality of clinical notes. The predecessor clinical note chronologically precedes the final clinical note. Referring to FIGS. 1 and 3A, for example, the method 200 may include selecting the predecessor clinical note 22 from the plurality of clinical notes 20.

At step 214, the method 200 further includes determining overlapping data between the final clinical note and the predecessor clinical note. Referring to FIGS. 3A and 3B, for example, the method 200 may include determining the overlapping data between the final clinical note 21 and the predecessor clinical note 22.

In some embodiments, a model determines the overlapping data between the final clinical note and the predecessor clinical note. Referring to FIGS. 1, 3A, and 3B, for example, the model 12 may determine the overlapping data between the final clinical note 21 and the predecessor clinical note 22.

In some embodiments, the determination of the overlapping data between the final clinical note and the predecessor clinical note is performed without access to historical electronic health record (EHR) data associated with the patient. Referring to FIGS. 1, 3A, and 3B, for example, the determination of the overlapping data between the final clinical note 21 and the predecessor clinical note 22 may be performed without access to the historical EHR data associated with the patient.

At step 215, the method 200 further includes identifying the overlapping data from the final clinical note. Referring to FIGS. 3A-3C, for example, the method 200 may include identifying the overlapping data from the final clinical note 21.

At step 216, the method 200 further includes performing an action on the identified overlapping data from the final clinical note to generate a synthesized input clinical note, such that the synthesized input clinical note is devoid of the overlapping data. Referring to FIGS. 3A-3C, for example, the method 200 may include performing an action on the identified overlapping data from the final clinical note 21 to generate the synthesized input clinical note 23.

In some embodiments, performing the action on the identified overlapping data may include removing the overlapping data from the final clinical note. For example, the method 200 may include removing the identified overlapping data from the final clinical note 21 to generate the synthesized input clinical note 23.

At step 217, the method 200 further includes providing the synthesized input clinical note and the predecessor clinical note to the machine learning model. Referring to FIGS. 1, 3A, and 3C, for example, the method 200 may include providing the synthesized input clinical note 23 and the predecessor clinical note 22 to the machine learning model 10.

At step 218, the method 200 further includes receiving a predicted final clinical note from the machine learning model. The machine learning model generates the predicted final clinical note based on the synthesized input clinical note and the predecessor clinical note. Referring to FIGS. 1 and 4, for example, the method 200 may include receiving the predicted final clinical note 25 from the machine learning model 10.

At step 219, the method 200 further includes updating the machine learning model based on the predicted final clinical note and the final clinical note. Referring to FIG. 1, 3B, and 4, for example, the method 200 may include updating the machine learning model 10 based on the predicted final clinical note 25 and the final clinical note 21.

At step 220, the method 200 may further include determining if all of the plurality of clinical notes 20 have been used to train the machine learning model 10. In other words, at step 220, the method 200 may include determining whether the training data (i.e., all of the plurality of clinical notes 20) is exhausted. If no, the method 200 may loop back to step 211, and another predecessor clinical note 22 and/or another final clinical note 21 may be used for further training of the machine learning model 10. If yes, the method 200 may proceed to step 221.

At step 221, the method 200 further includes outputting the machine learning model to at least one of a storage device or a non-transitory computer-readable storage. Referring to FIG. 1, for example, the method 200 may include outputting the machine learning model 10 to at least one of the storage device 130 or the non-transitory storage 110.

In some embodiments, the method 200 further includes embedding each of the plurality of clinical notes with clinical metadata prior to training the machine learning model. The clinical metadata includes one or more of a document type and a creation date. Referring to FIGS. 1 and 2, for example, the method 200 may include embedding each of the plurality of clinical notes 20 with the clinical metadata 20M prior to training the machine learning model 10.

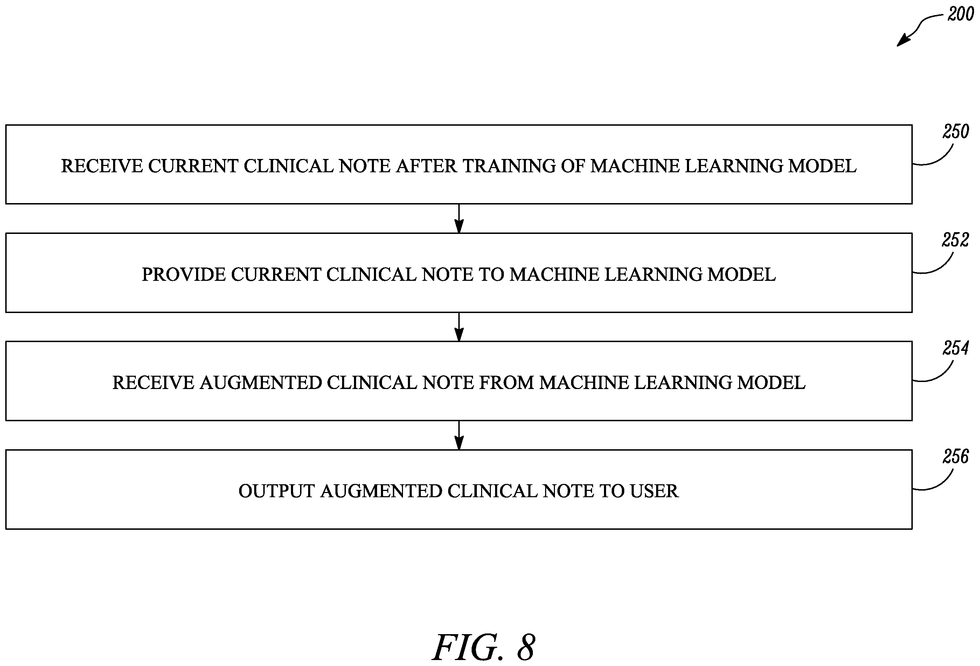

FIG. 8 illustrates a flowchart depicting further steps of the method 200 according to an embodiment of the present disclosure. In other words, steps 250-256 of the method 200 shown in FIG. 8 may be performed subsequent to the performance of steps 210-221 of the method 200 shown in FIG. 7. FIG. 8 will be described with further reference to FIGS. 1-6.

At step 250, the method 200 further includes receiving a current clinical note associated with the patient after training of the machine learning model. Referring to FIGS. 1 and 5, for example, the method 200 may include receiving the current clinical note 26 associated with the patient after training of the machine learning model 10.

In some embodiments, the current clinical note includes a transcription of a conversation between the patient and a user. Referring to FIG. 5, for example, the current clinical note 26 may include a transcription of the conversation (i.e., DoPaCo) between the patient and the user.

At step 252, the method 200 further includes providing the current clinical note to the machine learning model. Referring to FIGS. 1 and 5, for example, the method 200 may include providing the current clinical note 26 to the machine learning model 10.

At step 254, the method 200 further includes receiving an augmented clinical note from the machine learning model. The machine learning model generates the augmented clinical note based on the current clinical note. The augmented clinical note includes current data from the current clinical note and historical data associated with the patient that is not present in the current clinical note. Referring to FIGS. 1 and 6, for example, the method 200 may include receiving the augmented clinical note 27 from the machine learning model 10.

In some embodiments, the machine learning model further flags the historical data in the augmented clinical note for review by the user. Referring to FIGS. 1 and 6, for example, the machine learning model 10 may flag the historical data 27H in the augmented clinical note 27 for review by the user.

At step 256, the method 200 further includes outputting the augmented clinical note to the user. Referring to FIG. 6, for example, the method 200 may include outputting the augmented clinical note 27 to the user.

In some embodiments, the machine learning model modifies at least a portion of the current data. The machine learning model further flags the portion in the augmented clinical note for review by the user. Referring to FIGS. 1 and 6, for example, the machine learning model 10 may modify at least the portion 27P of the current data 26D, and the machine learning model 10 may flag the portion 27P in the augmented clinical note 27 for review by the user.

The method 200 may be used to train the machine learning model 10 using the plurality of clinical notes 20 associated with the patient to generate clinical notes, or more specifically, augmented clinical notes. Particularly, after training, the machine learning model 10 may receive the current clinical note 26 and generate the augmented clinical note 27 based on the current clinical note 26. That is, the plurality of clinical notes 20 and the current clinical note 26 may be inputs to the machine learning model 10, and the augmented clinical note 27 may be an output from the machine learning model 10.

The augmented clinical note 27 generated by the machine learning model 10 may include historical clinical data related to the patient that is not present in the current clinical note 26. In other words, the augmented clinical note 27 may include important clinical data related to the patient that is missing from the current clinical note 26. Specifically, the machine learning model 10 may learn to select appropriate historical clinical data from the plurality of clinical notes 20 to generate the augmented clinical note 27. In some examples, the machine learning model 10 may also modify (e.g., correct) clinical data of the current clinical note 26 based on the training, and output the modified clinical data (e.g., the portion 27P) in the augmented clinical note 27. The method 200 may therefore facilitate generation of clinical notes with more complete (e.g., historical information related to the patient) and correct information than what is present in the current clinical note 26.

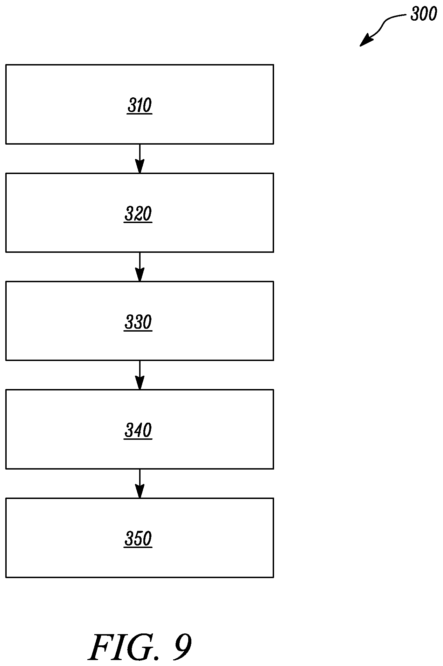

FIG. 9 illustrates a flowchart depicting a process 300 for training the machine learning model 10 (shown in FIG. 1) for generating clinical notes according to another embodiment of the present disclosure. In some embodiments, the process 300 may be performed by the processor 120 of the system 100 of FIG. 1. The process 300 will be generally described with further reference to FIG. 1.

At block 310, the process 300 may include receiving a source clinical note (SCN) associated with the patient. The SCN may be a clinical note that is generated based on the conversation between the patient and the user. In some embodiments, the SCN may be further based on admission/discharge/transfer (ADT) data associated with the patient. In some examples, the SCN may be generated by a person (e.g., a medical scribe) who does not have access to the EHR.

Optionally, the process 300 may include utilizing placeholders that indicate missing information for facilitating generation of the SCN. For example, placeholders such as: "patient is a <YEAR> old <GENDER> who presents shortness of breath," "Caloric restriction and regular physical activity advised for weight reduction," and "Target BMI is <BMI>" may be used to generate the SCN.

At block 320, the process 300 may further include generating an input clinical note (ICN) based on the SCN. Specifically, at block 320, the process 300 may include applying a series of pre-processing operations (e.g., text normalization, markup, etc.) to the SCN to generate the ICN.

At block 330, the process 300 may further include receiving the plurality of clinical notes 20 (alternatively referred to herein as "the PCN") associated with the patient. Each of the PCN chronologically precedes the ICN.

At block 340, the process 300 may include generating a final clinical note (FCN) based on the ICN and the PCN. The FCN may contain information (e.g., clinical data) from the ICN and the PCN. The FCN may be a training target for the machine learning model 10.

At block 350, the process 300 may include providing the ICN and one the PCN as inputs to the machine learning model 10, and the FCN as the training target to the machine learning model 10. Specifically, at block 350, the process 300 may include repetitively and iteratively providing the ICN and one of the PCN as the inputs to the machine learning model 10, and the FCN as the target to the machine learning model 10 until all of the PCN are exhausted.

The training of the machine learning model 10 may be improved by emphasizing specific information through additive embeddings. The additive embeddings may include marked up clinical concepts, embeddings to encode document type, embeddings representing how old an EHR document is in relation to the ICN, embeddings representing a code sets, etc. An example of this kind of information is codes from a code set (e.g., ICD-10, SNOMED, RxNORM) which can be represented with one or a combination of code-specific tokens, which are typically represented through trainable token embeddings.

At block 350, the process 300 may including generating, via the machine learning model 10, a predicted final clinical note (PFCN) based on the inputs (e.g., the ICN and the PCN) and the training target (e.g., the FCN).

In some embodiments, the machine learning model 10 may be an attention-based encoder-decoder transformer architecture that is capable of processing long input sequences. Specifically, in some embodiments, the machine learning model 10 may be a longformer-encoder-decoder (LED) model.

In some embodiments, the process 300 may further include providing data related to the user as additional training target to the machine learning model 10 (e.g., provided to the decoder of the LED model). The machine learning model 10 may therefore further learn to personalize the PFCN with respect to phrases and styles preferred by the user.

After training of the machine learning model 10 by the process 300, the machine learning model 10 may generate the PFCN upon receiving the ICN. In some embodiments, the machine learning model 10 may generate the PFCN upon receiving the SCN.

The process 300 may train the machine learning model 10 to generate improved clinical notes (i.e., the PFCN). The process 300 may be useful when a large number of the PCN is available for training the machine learning model 10.

It should be appreciated that the innovations disclosed herein are generally adapted to configure computer-based systems to address technical problems related to automatically generating clinical notes, and in particular, the technical problem of generating improved clinical notes based upon a doctor-patient conversation that include the important clinical information present in the historical clinical notes associated with the patient. That is, the innovations described herein are inextricably linked to computing systems that generate clinical notes from conversations (e.g., audio or transcription-based conversations). For the machine learning models disclosed herein to be sufficiently accurate, the number of parameters of the model must be large. For instance, the GPT-3 model utilizes a model with more than 100 billion parameters. In other words, using a GPT-3 model (or something similarly sized), each concept must be processed using more than 100 billion discrete calculations being performed in near real-time. Similarly, when training a machine learning model, the model must be trained in a forward pass of computations, and a backward pass of computations (known as backpropagation), until the model converges after a certain (but generally unknown) number of epochs (or iterations). As a result, even when the machine learning model to be trained according to this disclosure is fractionally the size of a large model (such as the GPT-3 model), training the model for its intended purpose as disclosed herein will nevertheless involve millions, or even billions, of calculations. In short, the sheer magnitude of the parameters and calculations involved for each concept takes the disclosed techniques outside the realm of what is practicable for a human to perform (either in the mind of a human or with the aid of pen and paper) and inextricably links the technology to the realm of computers.

Likewise, as is evident to one of ordinary skill in the art, the corpus of previous notes required to train or otherwise improve the machine learning models disclosed herein to extract relevant information makes it impracticable for a human to replace the computing systems disclosed herein to perform the techniques as described. Specifically, a human or group of humans could not produce a sufficient number of draft notes used to train the machine learning models disclosed herein in the time necessary to make such systems operable. To improve the model that can extract relevant information from previous notes and incorporate them in a draft clinical note a very large number of examples is required.

Additionally, it is not practicable-or even possible-for humans to identify information missing from previous visits to insert into the draft note in the time required by the system to make sure insertions are useful for its intended purpose. In particular, in a clinical setting, physicians and other medical providers need to review complete clinical notes within a few minutes of receiving the information: either during the in-person session with the patient or shortly thereafter. In other words, the timing requirements can only be satisfied using an automatic approach that analyzes the available previous notes and information, and determines how to transform the initial draft note to incorporate available but missing content as disclosed herein.

Unless otherwise indicated, all numbers expressing feature sizes, amounts, and physical properties used in the specification and claims are to be understood as being modified by the term "about." Accordingly, unless indicated to the contrary, the numerical parameters set forth in the foregoing specification and attached claims are approximations that can vary depending upon the desired properties sought to be obtained by those skilled in the art utilizing the teachings disclosed herein.

Although specific embodiments have been illustrated and described herein, it will be appreciated by those of ordinary skill in the art that a variety of alternate and/or equivalent implementations can be substituted for the specific embodiments shown and described without departing from the scope of the present disclosure. This application is intended to cover any adaptations or variations of the specific embodiments discussed herein. Therefore, it is intended that this disclosure be limited only by the claims and the equivalents thereof.

## Claims

1. A system comprising:
one or more computer processors; and
a non-transitory computer-readable storage having stored thereon instructions that when executed by the one or more processors cause the one or more processors to:
train a machine learning model to generate clinical notes by repeatedly:
receiving a plurality of clinical notes associated with a patient;
selecting a final clinical note from the plurality of clinical notes;
selecting a predecessor clinical note from the plurality of clinical notes, wherein the predecessor clinical note chronologically precedes the final clinical note;
determining overlapping data between the final clinical note and the predecessor clinical note;
identifying the overlapping data from the final clinical note;
performing an action on the identified overlapping data from the final clinical note to generate a synthesized input clinical note, such that the synthesized input clinical note is devoid of the overlapping data;
providing the synthesized input clinical note and the predecessor clinical note to the machine learning model;
receiving a predicted final clinical note from the machine learning model, wherein the machine learning model generates the predicted final clinical note based on the synthesized input clinical note and the predecessor clinical note;
updating the machine learning model based on the predicted final clinical note and the final clinical note; and
output the machine learning model to at least one of a storage device or the non-transitory computer-readable storage.

2. The system of claim 1, wherein each of the plurality of clinical notes is embedded with clinical metadata prior to training the machine learning model.

3. The system of any of claims 1-2, wherein the clinical metadata comprises one or more of a document type and a creation date.

4. The system of any of claims 1-3, wherein performing the action on the identified overlapping data comprises removing the identified overlapping data from the final clinical note.

5. The system of any of claims 1-4, wherein the determination of the overlapping data between the final clinical note and the predecessor clinical note is performed without access to historical electronic health record (EHR) data associated with the patient.

6. The system of any of claims 1-5, wherein a model determines the overlapping data between the final clinical note and the predecessor clinical note.

7. The system of any of claims 1-6, wherein the instructions when executed by the one or more processors further cause the one or more processors to:
receive a current clinical note associated with the patient after training of the machine learning model;
provide the current clinical note to the machine learning model;
receive an augmented clinical note from the machine learning model, wherein the machine learning model generates the augmented clinical note based on the current clinical note, and wherein the augmented clinical note comprises current data from the current clinical note and historical data associated with the patient that is not present in the current clinical note; and
output the augmented clinical note to a user.

8. The system of any of claims 1-7, wherein the machine learning model modifies at least a portion of the current data, and wherein the machine learning model further flags the portion in the augmented clinical note for review by the user.

9. The system of any of claims 1-8, wherein the machine learning model further flags the historical data in the augmented clinical note for review by the user.

10. The system of any of claims 1-9, wherein the current clinical note further comprises a transcription of a conversation between the patient and the user.

11. A computer-implemented method for using a machine learning model to generate clinical notes, the computer-implemented method comprising:
training the machine learning model to generate clinical notes by repeatedly:
receiving a plurality of clinical notes associated with a patient;
selecting a final clinical note from the plurality of clinical notes;
selecting a predecessor clinical note from the plurality of clinical notes, wherein the predecessor clinical note chronologically precedes the final clinical note;
determining overlapping data between the final clinical note and the predecessor clinical note;
identifying the overlapping data from the final clinical note;
performing an action on the identified overlapping data from the final clinical note to generate a synthesized input clinical note, such that the synthesized input clinical note is devoid of the overlapping data;
providing the synthesized input clinical note and the predecessor clinical note to the machine learning model;
receiving a predicted final clinical note from the machine learning model, wherein the machine learning model generates the predicted final clinical note based on the synthesized input clinical note and the predecessor clinical note;
updating the machine learning model based on the predicted final clinical note and the final clinical note; and
outputting the machine learning model to at least one of a storage device or a non-transitory computer-readable storage.

12. The computer-implemented method of claim 11, further comprising embedding each of the plurality of clinical notes with clinical metadata prior to training the machine learning model.

13. The computer-implemented method of any of claims 11-12, wherein the clinical metadata comprises one or more of a document type and a creation date.

14. The computer-implemented method of any of claims 11-13, wherein the determination of the overlapping data between the final clinical note and the predecessor clinical note is performed without access to historical electronic health record (EHR) data associated with the patient.

15. The computer-implemented method of any of claims 11-14, wherein a model determines the overlapping data between the final clinical note and the predecessor clinical note.
